(19) Europäisches Patentamt / European Patent Office / Office européen des brevets

(11) **EP 4 394 694 A1**

(12) **EUROPEAN PATENT APPLICATION**

(43) Date of publication:
**03.07.2024 Bulletin 2024/27**

(21) Application number: **22217379.1**

(22) Date of filing: **30.12.2022**

(51) International Patent Classification (IPC):
**G06T 7/00** $^{(2017.01)}$    **G16H 50/30** $^{(2018.01)}$

(52) Cooperative Patent Classification (CPC):
**G06T 7/0012; G16H 30/20; G16H 30/40;
G16H 50/20; G16H 50/30; G16H 50/50;**
G06T 2207/10072; G06T 2207/10132;
G06T 2207/30104

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Siemens Healthineers AG
91301 Forchheim (DE)**

(72) Inventors:
- **ITU, Lucian Mihai
  500294 Brasov (RO)**
- **CIMEN, Serkan
  Jersey City, 07310 (US)**

- **BERGER, Martin
  91088 Bubenreuth (DE)**
- **NEUMANN, Dominik
  91052 Erlangen (DE)**
- **TURCEA, Alexandru
  105500 Busteni, Prahova (RO)**
- **GULSUN, Mehmet Akif
  Princeton, 08540 (US)**
- **PASSERINI, Tiziano
  Plainsboro, 08536 (US)**
- **SHARMA, Puneet
  Princeton Junction, 08550 (US)**

(74) Representative: **Kraus & Lederer PartGmbB
Thomas-Wimmer-Ring 15
80539 München (DE)**

(54) **DETERMINATION OF HEMODYNAMIC INDICES**

(57) Techniques for processing multiple cardiac images are disclosed. The processing may take place either during or after an angiography exam of a coronary artery of interest. The multiple cardiac images are obtained either during or after the angiography exam. Each of the multiple cardiac images depicts a respective segment of the coronary artery of interest (3100). A geometric structure of the coronary artery of interest is determined based on the multiple cardiac images (3200). A lumped parameter model of the coronary artery of interest is determined based on the geometric structure (3300), and respective values of at least one hemodynamic index at a position of the coronary artery of interest is determined based on the lumped parameter model of the coronary artery of interest (3400).

FIG 3

~3000

obtaining multiple cardiac images, each of the multiple cardiac images depicting a respective segment of a coronary artery of interest ~3100

determining a geometric structure of the coronary artery of interest based on the multiple cardiac images ~3200

determining, based on the geometric structure, a lumped parameter model of the coronary artery of interest ~3300

determining, based on the lumped parameter model of the coronary artery of interest, respective values of at least one hemodynamic index at a position of the coronary artery of interest ~3400

EP 4 394 694 A1

**Description**

TECHNICAL FIELD

Various examples of the disclosure generally relate to angiography. Various examples specifically relate to processing multiple cardiac images depicting a coronary artery of interest to determine respective values of at least one hemodynamic index at a position of the coronary artery of interest.

BACKGROUND

The gold standard for the functional assessment of coronary artery stenosis is the Fractional Flow Reserve (FFR) index. FFR is a hemodynamic index to determine the hemodynamic significance of a given epicardial coronary stenosis. It can be defined as the ratio of maximal achievable blood flow in a myocardial bed in the presence of an epicardial stenosis to the theoretical normal maximal flow in the same myocardial distribution. However, FFR is measured invasively with the help of such as angiography. During such an invasive procedure, a contrast agent is injected through a catheter and into coronary arteries to make them easier to see. However, exposure to the contrast agent may cause kidney diseases or allergic reactions. Additionally, for an accurate measurement, FFR is measured when blood flow is at its highest, and thereby certain medicine, e.g., adenosine or papaverine is used to increase the blood flow. Such medicine may incur health risks.

To overcome drawbacks of invasive FFR measurements, newer technologies such as using either computational fluid dynamics (CFD) or machine learning (ML), a virtual FFR can be derived from X-ray Angiography or computed tomography. For example, non-patent literature - Tröbs, Monique, et al.

"Comparison of fractional flow reserve based on computational fluid dynamics modeling using coronary angiographic vessel morphology versus invasively measured fractional flow reserve." The American journal of cardiology 117.1 (2016): 29-35. - discloses a new approach to determine lesion-specific FFR on the basis of coronary anatomy as visualized by invasive coronary angiography.

Similarly, other hemodynamic indices like rest diastolic pressure ratio (dPR), instantaneous wave-free ratio (iFR), etc. may also be of interest and measured invasively or derived from either X-ray Angiography or computed tomography. However, such methods for computing FFR or other hemodynamic indices focus on a region of interest that is visible in a single angiographic frame.

Under certain circumstances, a region of interest for the computation of the hemodynamic index (e.g., those indices outlined above) may not be entirely visible or not well visible in a single angiographic frame. For example, typically when the region of interest, e.g., the entire right coronary artery (RCA) or the entire left coronary artery (LCA), is comparatively large, the angiographic acquisition displays on the first frame the proximal part of the coronary tree, and then, after panning, the more distal part of the coronary tree is displayed on the later frames. Further, depending on the angulation of the acquisition, the entire region of interest may not be well visible on a single frame/acquisition. Typically, different angulations are recommended for good visualization of the proximal /mid /distal region of a coronary vessel tree. In some examples, a coronary tree is not entirely in the field of view. The coronary arteries may display significant motion, which may lead to situations where part of the coronary tree moves out of the field of view, e.g., encountered in the RCA views. In some further examples, the duration of contrast agent injection is very short. If the contrast agent is injected for a very short time this may lead to an acquisition where no frame can be found on which all coronary arteries of interest, i.e., the entire region of interest, are well visible. Consequently, the proximal part of the region of interest may be well visible in an early frame, and the distal part of the region of interest may be well visible in a later frame.

Consequently, the available methods in the prior art cannot be applied under such circumstances that the region of interest for the computation/determination of the hemodynamic index is not entirely visible or not well visible in a single angiographic frame.

SUMMARY

Therefore, a need exists for advanced techniques for determining one or more hemodynamic index. Specifically, a need exists for advanced techniques of determining one or more hemodynamic index for a large region of interest. A need exists for precisely determining one or more hemodynamic index for a specific patient.

This need is met by the features of the independent claims. The features of the dependent claims define embodiments.

A computer-implemented method for processing multiple cardiac images is provided. The method comprises obtaining multiple cardiac images. Each of the multiple cardiac images depicts a respective segment of a coronary artery of interest. The method further comprises determining a geometric structure of the coronary artery of interest based on the multiple cardiac images and determining, based on the geometric structure, a lumped parameter model of the coronary artery of interest. The method also comprises determining, based on the lumped parameter model of the coronary artery of interest, respective values of at least one hemodynamic index at a position of the coronary artery of interest.

According to various examples, the multiple cardiac images may comprise multiple frames of an angiogram acquired using different angulations.

According to various examples, the at least one hemodynamic index may comprise at least one of a blood pressure, a blood flow rate, a fractional flow reserve, a heart rate, a cardiac index, a mean arterial pressure, a systemic vascular resistance index, a central venous pressure, and a central venous oxygen saturation.

A computing device comprising a processor and a memory is provided. Upon loading and executing program code from the memory, the processor is configured to perform a method for processing multiple cardiac images. The method comprises obtaining multiple cardiac images. Each of the multiple cardiac images depicts a respective segment of a coronary artery of interest. The method further comprises determining a geometric structure of the coronary artery of interest based on the multiple cardiac images and determining, based on the geometric structure, a lumped parameter model of the coronary artery of interest. The method also comprises determining, based on the lumped parameter model of the coronary artery of interest, respective values of at least one hemodynamic index at a position of the coronary artery of interest.

An angiography device comprising a computing device is provided. The computing device comprises a processor and a memory. Upon loading and executing program code from the memory, the processor is configured to perform a method for processing multiple cardiac images. The method comprises obtaining multiple cardiac images. Each of the multiple cardiac images depicts a respective segment of a coronary artery of interest. The method further comprises determining a geometric structure of the coronary artery of interest based on the multiple cardiac images and determining, based on the geometric structure, a lumped parameter model of the coronary artery of interest. The method also comprises determining, based on the lumped parameter model of the coronary artery of interest, respective values of at least one hemodynamic index at a position of the coronary artery of interest.

A computer program product or a computer program or a computer-readable storage medium including program code is provided. The program code can be executed by at least one processor. Executing the program code causes the at least one processor to perform a method for processing multiple cardiac images. The method comprises obtaining multiple cardiac images. Each of the multiple cardiac images depicts a respective segment of a coronary artery of interest. The method further comprises determining a geometric structure of the coronary artery of interest based on the multiple cardiac images and determining, based on the geometric structure, a lumped parameter model of the coronary artery of interest. The method also comprises determining, based on the lumped parameter model of the coronary artery of interest, respective values of at least one hemodynamic index at a position of the coronary artery of interest.

It is to be understood that the features mentioned above and those yet to be explained below may be used not only in the respective combinations indicated, but also in other combinations or in isolation without departing from the scope of the disclosure.

BRIEF DESCRIPTION OF THE DRAWINGS

FIG. 1 schematically illustrates aspects with respect to a C-arm machine.

FIG. 2 schematically illustrates an exemplary 3D frontal view of a heart and of coronary arteries.

FIG. 3 is a flowchart of a method according to various examples.

FIG. 4 schematically illustrates two exemplary frames of an angiogram according to various examples.

FIG. 5 schematically illustrates an exemplary procedure for determining a geometric structure of a coronary artery of interest according to various examples.

FIG. 6 schematically illustrates an exemplary lumped parameter model of a coronary artery of interest according to various examples.

FIG. 7 schematically illustrates a further exemplary lumped parameter model of a coronary artery of interest according to various examples.

FIG. 8 schematically illustrates a still further exemplary lumped parameter model of a coronary artery of interest according to various examples.

FIG. 9 is a block diagram of a computing device according to various examples.

DETAILED DESCRIPTION OF THE DRAWINGS

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the disclosure will be described in detail with reference to the accompanying drawings. It is to be understood that the following description of embodiments is not to be taken in a limiting sense. The scope of the disclosure is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques for determining a respective value of at least one hemodynamic index are described. The at least one hemodynamic index may comprise at least one of a blood pressure, a blood flow rate, a fractional flow reserve, a heart rate, a cardiac index, a mean arterial pressure, a systemic vascular resistance index, a central venous pressure, and a central venous oxygen saturation.

According to this disclosure, techniques for processing multiple cardiac images, each of which depicts a respective segment of a coronary artery of interest, are disclosed.

The multiple cardiac images may be obtained either during or after an angiography exam of the coronary artery of interest. The processing may take place either during or after the angiography exam of the coronary artery of interest, i.e., either real-time processing or post-processing is possible.

The multiple cardiac images, each of which depicts a respective segment of the coronary artery of interest, are obtained either during or after an angiography exam. A geometric structure of the coronary artery of interest is determined based on the multiple cardiac images. A lumped parameter model of the coronary artery of interest is determined based on the geometric structure, and respective values of at least one hemodynamic index at a position of the coronary artery of interest is determined based on the lumped parameter model of the coronary artery of interest.

According to various examples, the multiple cardiac images may be obtained directly from an angiography device or from a database for storing the multiple cardiac images acquired by the angiography device, e.g., a picture archiving and communication system (PACS).

According to various examples, the respective values of the at least one hemodynamic index at the position of the coronary artery of interest may be more precisely determined by taking into account at least one measurement associated with the at least one hemodynamic index at a further position within a region of the coronary artery of interest arranged upstream with respect to the position. The at least one measurement associated with the at least one hemodynamic index at the further position may be obtained using a pressure wire. The pressure wire has a pressure sensor near the end and the further position may be located using an angiography device. Alternatively, the at least one measurement associated with the at least one hemodynamic index at the further position may be obtained from a database, e.g., the PACS. For example, each of the at least one measurement may be stored together with a respective cardiac image comprising the further position.

According to various examples, the angiography device may comprise a C-arm X-ray machine, an intravascular ultrasound machine, an optical coherence tomography machine, or a coronary computed tomography angiography machine.

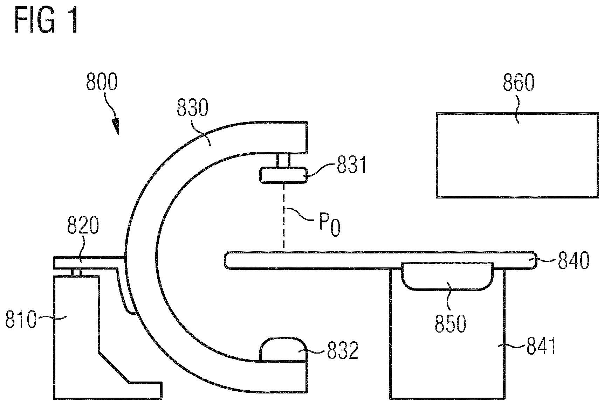

As an exemplary angiography device, FIG. 1 schematically illustrates aspects with respect to a C-arm machine 800. The C-arm machine 800 may be used for both angiography and fluoroscopy. In FIG. 1, the C-arm machine 800 is in neutral position $P_0$. It is possible to manipulate the C-arm machine 800 to be in a rotated position deviating from the neutral position $P_0$. The angle between the neutral position P0 and a specific rotated position is referred to as the angiography angle or the fluoroscopy angle. The C-arm machine 800 comprises a rotatable C arm 830 on which X-ray emission means 831 and X-ray detection means 832 may be mounted. The C arm 830 and thereby X-ray emission means 831 and X-ray detection means 832 are positioned to center around patient surface 840. X-ray emission means 831 may emit X-rays which may penetrate through a patient positioned on the patient surface 840. X-ray detection means 832 detects the X-rays emitted from X-ray emission means 831. When a patient on the patient surface 840 is injected with a radio-opaque contrast agent into the patient's vessels, some of the X-rays emitted by X-ray emission means 831 are absorbed by the radio-opaque contrast agent, leading X-ray detection means 832 to detect a sequence of images of the vessels filled with the radio-opaque contrast agent, i.e. an angiogram. X-ray emission means 831 and X-ray detection means 832 may also collectively be referred to as x-ray imaging means.

The C arm 830 may be coupled to a C arm rotation unit 820. The C arm rotation unit 820 may be any motorized means configured to rotate the C arm 830 according to an angiography angel or a fluoroscopy angle. The C arm rotation unit 820 may be attached to and controlled by a C arm control until 810. The C arm control unit 810 may be any kind of circuitry capable of controlling C arm 830. For example, the C arm control unit 810 may include a computing device.

The C-arm machine 800 may further include a control panel 850 mounted onto a side surface of patient surface support 841. The control panel 850 may be used to control C arm 830 in order to guide medical specialists to pathological vessels. Fig. 1 does not show any connections between control panel 850 and C arm 830 to simplify the depiction of the exemplary C-arm machine 800. In some examples, the connection may be wireless. In some further examples, the connection may be wired and may e.g., be integrated into the ceiling of the room where the C-arm machine 800 is located.

The C-arm machine 800 may also include a display 860. The display 860 may be used to display information to the medical specialist, such as real-time fluoroscopy image with an overlaid vessel roadmap image including a path to one or more pathological vessels, and/or a location of the pressure wire for measuring the at least one measurement associated with the at least one hemodynamic index. Further, the display 860 may be used to display vessel segmentation data included in overlaid vessel roadmap images, including labels for various vessel segments. In some examples, display 860 may be a touch screen, which may be used to toggle the display of the vessel segmentation data on and off.

The C-arm machine 800 may be connectable to a database (not shown in FIG. 1), such as a PACS located within a local network of a hospital, for storing angiograms and/or measurements associated with the at least one hemodynamic index.

According to various examples, a name (e.g., any one in the column "Abbreviation" in Table 1) of a specific segment of a coronary artery of interest or of the entire coronary artery of interest can be determined based on a region of interest of the angiography exam. For example, such a region of interest may comprise the left or right branch of the coronary vessel tree, or a specific vessel section. The region of interest may be determined based on user input, or on a patient's electronic medical record included in a health information system.

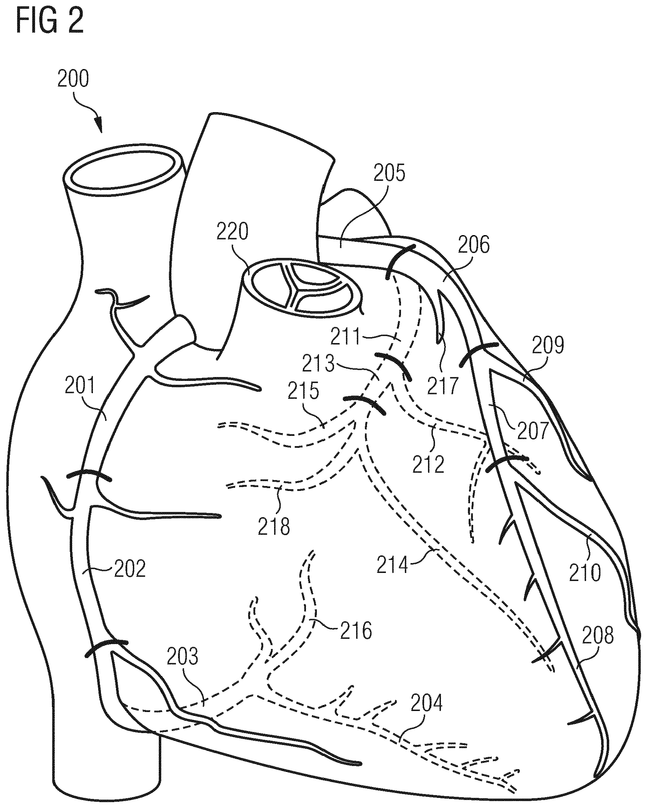

For example, the coronary artery of interest within the region of interest may be one or more segments of the coronary arteries of the heart. FIG. 2 schematically illustrates an exemplary 3D frontal view 200 of a heart and of coronary arteries. The segments of the coronary arteries in FIG. 2 are based on and numbered according to the vessel segmentation as proposed by the American Heart Association (AHA) and as amended by the Society of Cardiovascular Computed Tomography (SCCT). Table 1 provides a short description of the respective vessel segments as well as an abbreviation for each vessel segment.

*Table 1: Segments of the Coronary Arteries in FIG. 2.*

| Ref. Sign | Vessel Segment | Abbreviation | Description |
|---|---|---|---|
| 201 | proximal right coronary artery | pRCA | Ostium of the RCA to one-half the distance to the acute margin of heart |
| 202 | Mid RCA | mRCA | End of pRCA to the acute margin of heart |
| 203 | Distal RCA | dRCA | End of mRCA to origin of the PDA (posterior descending artery) |
| 204 | PDA-R | R-PDA | PDA from RCA |
| 205 | Left main | LM | Ostium of LM to bifurcation of LAD (left anterior descending artery) and LCx (left circumflex artery) |

(continued)

| Ref. Sign | Vessel Segment | Abbreviation | Description |
|---|---|---|---|
| 206 | Proximal LAD | pLAD | End of LM to the first large septal or D1 (first diagonal), whichever is most proximal |
| 207 | Mid LAD | mLAD | End of proximal LAD to one-half the distance to the apex |
| 208 | Distal LAD | dLAD | End of mid LAD to end of LAD |
| 209 | D1 | D1 | First diagonal branch D1 |
| 210 | D2 | D2 | Second diagonal branch D2 |
| 211 | Proximal LCx | pCx | End of LM to the origin of the OM1 (first obtuse marginal) |
| 212 | OM1 | OM1 | First OM1 traversing the lateral wall of the left ventricle |
| 213 | Mid and distal LCx | LCx | Traveling in the atrioventricular groove, distal to the OM1 branch to the end of the vessel or origin of the L-PDA |
| 214 | OM2 | OM2 | Second marginal OM2 |
| 215 | PDA-L | L-PDA | PDA from LCx |
| 216 | PLB-R | R-PLB | PLB from RCA |
| 217 | Ramus intermedius | RI | Vessel originating from the left main between the LAD and LCx in case of a trifurcation |
| 218 | PLB-L | L-PLB | PLB from LCx |

In addition to the vessel segments listed in Table 1, FIG. 2 also indicates the aortic valve 220.

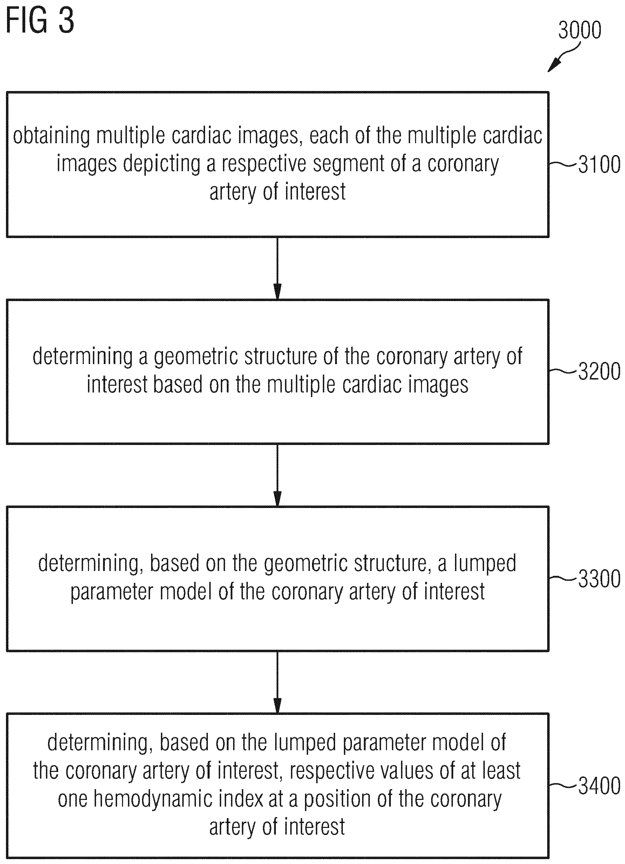

FIG. 3 is a flowchart of a method 3000 according to various examples. For example, the method 3000 according to FIG. 3 may be executed by the C arm control unit 810 of the C-arm machine 800 according to the example of FIG. 1, e.g., upon loading program code from a memory. The method 3000 may be executed by the C arm control unit 810 together with the control panel 850, for example, the C arm control unit 810 and the control panel 850 may be together configured to acquire one or more cardiac images, e.g., angiograms, during an angiography exam of a region of interest comprising a coronary artery of interest and the C arm control unit 810 may be further configured to process one or more cardiac images, e.g., one or more frames of the acquired angiograms. Further, the method 3000 may be executed by the C arm control unit 810 itself. It would also be possible that the method 3000 is at least partially executed by a separate compute unit, e.g., at a server backend.

FIG. 3 illustrates aspects with respect to processing multiple cardiac images, each of which depicts a respective segment of a coronary artery of interest (e.g., LAD (i.e., a combination of pLAD 206, mLAD 207, and dLAD 208), or RCA (i.e., a combination of pRCA 201, mRCA 202, and dRCA 203) as shown in FIG. 2). The multiple cardiac images are obtained either during or after an angiography exam and thereby the processing can be either real-time processing or post-processing. A geometric structure of the coronary artery of interest is determined based on the multiple cardiac images. A geometric structure of the coronary artery of interest is determined based on the multiple cardiac images. A lumped parameter model of the coronary artery of interest is determined based on the geometric structure, and respective values of at least one hemodynamic index at a position of the coronary artery of interest is determined based on the lumped parameter model of the coronary artery of interest. Details of the method 3000 are described below.

Block 3100: obtaining multiple cardiac images, each of the multiple cardiac images depicting a respective segment of a coronary artery of interest.

For example, the multiple cardiac images may be obtained directly from an angiography device, e.g., the C-arm machine 800 of FIG. 1, or from a database for storing the multiple cardiac images acquired by the angiography device, e.g., a PACS. Alternatively, the multiple cardiac images may be obtained from imaging data acquired using computed tomography angiography.

Hereinafter, techniques will be explained using frames of X-ray angiograms as an example of cardiac images. I.e., a single cardiac image may correspond to a frame of an angiogram.

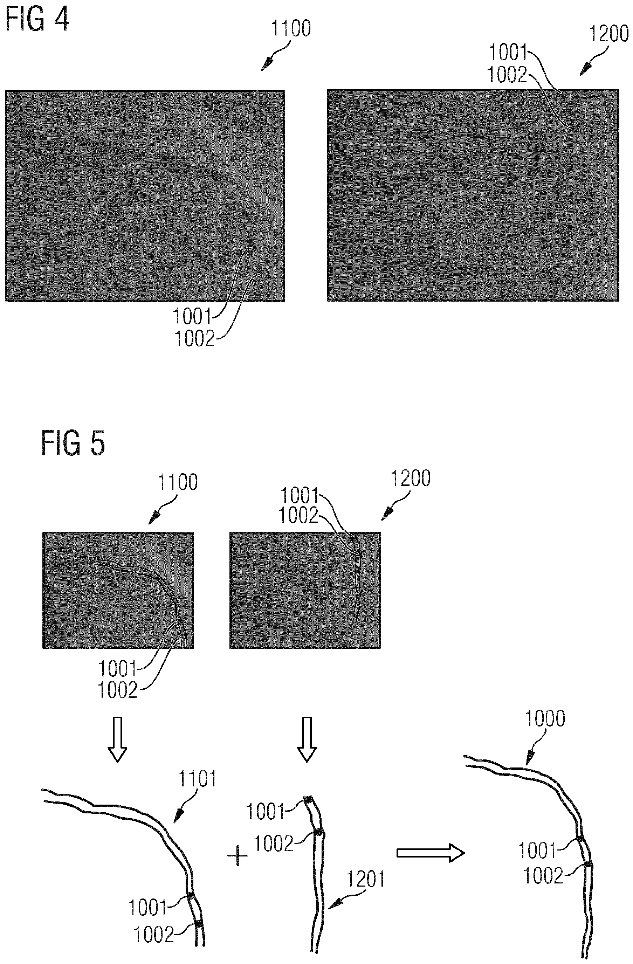

According to various examples, the region of interest comprising the coronary artery of interest may be too large to be imaged in a single cardiac image, e.g., in a single frame of an angiogram. I.e., different parts of the region of interest may be visible in different frames of the angiogram. For example, FIG. 4 shows two frames 1100 and 1200 of an angiogram acquired during an angiography exam of a coronary artery of interest, i.e., the LAD (i.e., a combination of pLAD 206, mLAD 207, and dLAD 208). The pLAD 206 and the mLAD 207 are visible in the frame 1100, while the mLAD 207 and the dLAD 208 are visible in the frame 1200. The frame 1100 may be acquired earlier than the frame 1200 and the frame 1200 may be acquired after panning.

To improve the accuracy of determination of respective values of the at least one hemodynamic index at a position, it is possible to automatically and precisely select or determine, among frames of an angiogram, multiple appropriate frames, e.g., the frames 1100 and 1200, where an upstream part (typically an early frame) and a downstream part (typically a later frame) of the LAD are visible.

According to various examples, said obtaining of the multiple cardiac images may comprise determining, based on at least one frame at the beginning of an angiogram, at least one landmark associated with the coronary artery of interest; determining, among frames of the angiogram, a frame at which panning begins and a further frame at which the panning ends based on the at least one landmark; selecting, among the frames of the angiogram, the multiple cardiac images based on the frame and the further frame such that the multiple cardiac images pertain to the same cardiac phase and depicts the coronary artery of interest.

For example, as shown in FIG. 4, two landmarks 1001 and 1002, i.e., bifurcations, may be determined based on the frame 1100. It is also possible to select stenosis or other background structures that are visible on different frames as landmarks. The frame at which panning begins may be determined by tracking at least one of the landmarks 1001 and 1002 and based on a predefined threshold of a change of respective positions of the landmarks 1001 and 1002. I.e., once the landmarks 1001 and/or 1002 have changed their locations beyond the predefined threshold, panning may be considered to have started. Similarly, the further frame at which the panning ends can be determined by further tracking the landmarks 1001 and 1002. Once the respective position of the landmarks 1001 and/or 1002 remains approximately constant, panning may be considered to have stopped. Alternatively, if panning does not stop, a frame can be selected where the landmarks are still visible in the image (in case of significant panning the landmarks may leave the field of view). Finally, the multiple cardiac images (or frames), e.g., the frames 1100 and 1200, may be selected based on vesselness information, cardiac phase information, and/or start and end frames for panning. The cardiac phase information may be determined using techniques as disclosed in a US patent with patent number US 11, 051, 779 B2.

Additionally or optionally, the selection of the multiple appropriate frames, e.g., the frames 1100 and 1200, may be performed such that the selected frames are well contrasted, pertain to a certain cardiac phase (typically end-diastole is desired), and display both the upstream and downstream parts of the coronary artery of interest.

Alternatively, the selection of multiple appropriate frames may be performed semi-automatically or fully manually. For example, the multiple appropriate frames may be selected by experts when acquiring an angiogram or viewing the angiogram.

Block 3200: determining a geometric structure of the coronary artery of interest based on the multiple cardiac images.

For example, the geometric structure of the coronary artery of interest may be determined by segmenting the coronary artery of interest from the multiple cardiac images.

According to various examples, said determining of the geometric structure of the coronary artery of interest may comprise determining, for each of the multiple cardiac images, a respective segment of the geometric structure of the coronary artery of interest based on the respective one of the multiple cardiac images and merging the respective segments of the geometric structure of the coronary artery of interest into the geometric structure of the coronary artery of interest.

FIG. 5 schematically illustrates an exemplary procedure for determining a geometric structure of a coronary artery of interest according to various examples. As shown in FIG. 5, two segments 1101 and 1201 of the geometric structure of the coronary artery of interest may be respectively determined based on the two frames 1100 and 1200. Then, the two segments 1101 and 1201 may be merged into the geometric structure of the coronary artery of interest 1000, e.g., based on at least one of the two landmarks 1001 and 1002.

Alternatively, the geometric structure of the coronary artery of interest may be determined in other approaches. For example, the method 3000 may further comprise merging the multiple cardiac images into a merged cardiac image and said determining of the geometric structure of the coronary artery of interest is based on the merged cardiac image.

Referring to FIG. 5, it is also possible that the two frames 1100 and 1200 are merged into a merged cardiac image, e.g., based on at least one of the two landmarks 1001 and 1002. Then, the geometric structure of the coronary artery of interest 1000 may be determined by segmenting the coronary artery of interest from the merged cardiac image.

Alternatively or optionally, various techniques for determining a 2D/3D/4D geometric structure of the coronary artery are available in the prior art. For example, non-patent literature - Çimen, Serkan, et al. "Reconstruction of coronary arteries from X-ray angiography: A review." Medical image analysis 32 (2016): 46-68. - reviews various methods of reconstruction of coronary arteries from X-ray angiography. In addition, non-patent literature - Andriotis, Adamantios, et al. "A new method of three-dimensional coronary artery reconstruction from X-ray angiography: Validation against a virtual phantom and multislice computed tomography." Catheterization and cardiovascular interventions 71.1 (2008): 28-43. - discloses a method of three-dimensional coronary artery reconstruction from X-ray angiography.

Block 3300: determining, based on the geometric structure of the coronary artery of interest, a lumped parameter model of the coronary artery of interest.

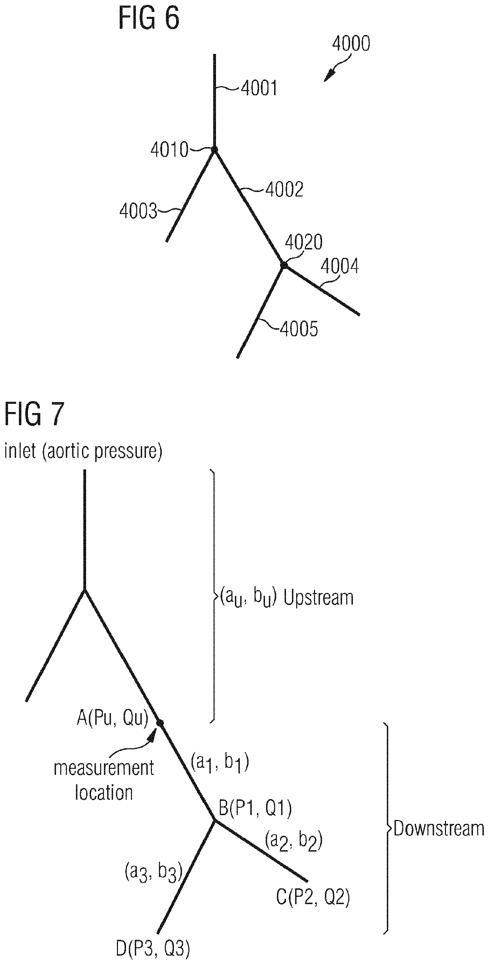

FIG. 6 schematically illustrates an exemplary lumped parameter model 4000 of a coronary artery of interest according to various examples. The lumped parameter model 4000 comprises five segments 4001-4005 of the coronary artery of interest and two bifurcations 4010 and 4020 delimiting respective segments of the five segments 4001-4005. Each of the five segments 4001-4005 may be described or modeled by a pair of coefficients characterizing a total energy loss of blood flow passing from a respective inlet of the corresponding segment to a respective outlet of the corresponding segment. The total energy loss may comprise various energy losses, e.g., viscous losses, convective losses, and turbulent losses, incurred by aspects like tortuosity, stenosis eccentricity, positive remodeling, etc.

According to this disclosure, to obtain the values of each pair of the coefficients for each segment of the lumped parameter model 4000, a hemodynamic model (e.g., 0D / 1D / 3D / 3D fluid-structure interactions) may be employed, which may take as input the geometric structure of a respective segment of the coronary artery of interest.

Additionally or optionally, different flow rate values (Q) may be considered for running a large number of computations, to obtain a precise estimation of the respective pair of the coefficient.

According to various examples, each of five segments 4001-4005 may be also defined based on healthy/stenotic status. As such, each segment may be described or modeled by one or more pairs of coefficients.

Block 3400: determining, based on the lumped parameter model of the coronary artery of interest, respective values of at least one hemodynamic index at a position of the coronary artery of interest.

For example, referring to FIG. 6, respective pressure drop of each of the five segments 4001-4005 may be determined using the following equation:

$$\Delta P_i = a_i * Q_i + b_i Q_i^2$$

Wherein index i refers to the respective segment, e.g., any of the five segments 4001-4005; $Q_i$ refers to a flow rate at respective inlet of each of the five segments 4001-4005, and $(a_i, b_i)$ is the respective pair of coefficients characterizing a total energy loss of blood flow passing from a respective inlet of the corresponding segment to a respective outlet of the corresponding segment.

Accordingly, the respective pressure drop and/or flow rate may be determined in an iterative manner starting from the root of the aorta.

Alternatively, according to various examples, the respective values of the at least one hemodynamic index at the position of the coronary artery of interest may be determined using a machine-learning-based approach. The machine-learning-based approach may take as input the aortic pressure, i.e., the blood pressure at the root of the aorta and the respective pair of coefficients. Such a machine-learning-based approach may be as disclosed in non-patent literature - Itu, Lucian, et al. "A machine-learning approach for computation of fractional flow reserve from coronary computed tomography." Journal of applied physiology 121.1 (2016): 42-52.

According to various examples, the respective values of the at least one hemodynamic index at the position of the coronary artery of interest may be more precisely determined by taking into account at least one measurement associated with the at least one hemodynamic index at a further position within a region of the coronary artery of interest arranged upstream with respect to the position. The method 3000 may further comprise obtaining at least one measurement associated with the at least one hemodynamic index at a further position within a region of the coronary artery of interest arranged upstream with respect to the position, and said determining of the respective values of the at least one hemodynamic index at the position is further based on the at least one measurement at the further position.

According to various examples, the at least one measurement associated with the at least one hemodynamic index at the further position may be obtained using a pressure wire. The pressure wire has a pressure sensor near the end and the first position may be located using an angiography device, e.g., the C-arm machine 800 of FIG. 1.

Alternatively, the at least one measurement associated with the at least one hemodynamic index at the further position may be obtained from a database, e.g., the PACS.

Additionally or optionally, the at least one measurement may comprise measurement obtained/derived using at least one of the following imaging machines: a C-arm X-ray machine, an intravascular ultrasound (IVUS) machine, an optical coherence tomography (OCT) machine, and a coronary computed tomography angiography (CCTA) machine.

For example, the at least one measurement may comprise measurement derived from previously computed/determined hemodynamic values including from other modalities, e.g., hemodynamics are already simulated on CCTA, IVUS, OCT data. The existing virtual hemodynamic measurements may be registered to angiography, e.g., to extend them to a broader coverage by including more proximal or distal vessel parts.

According to various examples, the at least one hemodynamic index may comprise a blood pressure and a blood flow rate, and said determining of the respective values of the at least one hemodynamic index at the position may comprise determining, based on the at least one measurement, both the blood pressure and the blood flow rate at the further position of the coronary artery of interest; determining, using the lumped parameter model of the coronary artery of interest, coefficients characterizing a total energy loss of blood flow passing from the further position to the position based on the geometric structure of a segment of the coronary artery of interest between the further position and the position; determining, based on both the blood pressure and the blood flow rate at the further position of the coronary artery of interest and the coefficients, the respective values of the at least one hemodynamic index at the position.

According to various examples, both the blood pressure and the blood flow rate at the further position of the coronary artery of interest may be determined according to the following three scenarios. The further position and the position respectively correspond to the first position and the second position herein.

Scenario 1:

Both the blood pressure and the blood flow rate at the first(further) position of the coronary artery of interest may be directly measured, i.e., the at least one measurement may comprise both the blood pressure and the blood flow rate at the first/further position of the coronary artery of interest.

Scenario 2: The entire segment of the coronary artery of interest upstream from the measurement location is visible.

The at least one measurement may comprise a blood pressure drop between the root of the aorta and the first position, and the geometric structure of the coronary artery of interest comprises a further segment of the coronary artery of interest between the root of the aorta and the first position. Said determining of both the blood pressure and the blood flow rate at the first position of the coronary artery of interest may comprise obtaining the central aortic blood pressure at the root of the aorta; determining, using the lumped parameter model of the coronary artery of interest, further coefficients characterizing a further total energy loss of blood flow passing from the root of the aorta to the first position based on the geometric structure of the further segment of the coronary artery of interest between the root of the aorta and the first position; determining, based on the blood pressure drop, the central aortic blood pressure, and the further coefficients, both the blood pressure and the blood flow rate at the first position of the coronary artery of interest.

FIG. 7 schematically illustrates a further exemplary lumped parameter model of a coronary artery of interest according to various examples. As shown in FIG. 7, point A is a measurement location, i.e., the first position. $P_u$ and $Q_u$ are respectively pressure and flow rate at point A and may be respectively derived based on the measured blood pressure drop $\Delta P_u$ between the root of the aorta and the first position and the measured aortic pressure at the inlet using the following equations:

$$\Delta P_u = a_u * Q_u + b_u Q_u^2 \qquad (1)$$

$$P_u = aortic\ pressure - \Delta P_u \qquad (2)$$

$(a_u, b_u)$ is the pair of further coefficients characterizing the further total energy loss of the blood flow passing from the root of the aorta to the first position, i.e., the upstream of the first position.

After determining the pressure and flow rate at point A, i.e., the first position, the respective values of pressure and flow rate at a second position within a region of the coronary artery of interest arranged downstream with respect to the first position can be determined according to the following description.

Point B is the intersection between the two branches BD and BC. $P_1$ and $Q_1$ are respectively pressure and flow rate at point B and may be respectively derived using the following equation:

$$\Delta P_1 = P_u - P_1 = a_1 * Q_1 + b_1 * Q_1^2 \qquad (3)$$

$(a_1, b_1)$ is the pair of coefficients characterizing the total energy loss of the blood flow passing from point A to point B.

Point C is the distal end of the branch BC. $P_2$ and $Q_2$ are respectively pressure and flow rate at point C and may be respectively derived using the following equation:

$$\Delta P_2 = P_1 - P_2 = a_2 * Q_2 + b_2 * Q_2^2 \qquad (4)$$

$(a_2, b_2)$ is the pair of coefficients characterizing the total energy loss of the blood flow passing from point B to point C.

Point D is the distal end of the branch BD. $P_3$ and $Q_3$ are respectively pressure and flow rate at point D and may be respectively derived using the following equation:

$$\Delta P_3 = P_1 - P_3 = a_3 * Q_3 + b_3 * Q_3^2 \qquad (5)$$

$(a_3, b_3)$ is the pair of coefficients characterizing the total energy loss of the blood flow passing from point B to point D.

$(a_u, b_u)$, $(a_1, b_1)$, $(a_2, b_2)$, and $(a_3, b_3)$ can be respectively determined as outlined above. In addition, $Q_1 = Q_u$, and $Q_1 = Q_2 + Q_3$. Additionally, there are microvascular resistances at each outlet, each of them adding one equation. The pressure after the microvascular resistance is zero. All equations (3)-(5) are solved in an iterative process.

As such, the respective pressure and flow rate at each of points B, C, and D can be determined iteratively. Each of points B, C, and D may be the second position within a region of the coronary artery of interest arranged downstream with respect to the first position. The second position may be any position located in any of the branches AB, BC, and BD.

Scenario 3: The entire segment of the coronary artery of interest upstream from the measurement location is partially or completely missing.

Said determining of both the blood pressure and the blood flow rate at the first position of the coronary artery of interest may comprise obtaining the central aortic blood pressure at the root of the aorta, wherein the at least one measurement comprises an FFR measured at the first position and relative to the central aortic blood pressure; and determining, based on the central aortic blood pressure, and the FFR, both the blood pressure and the blood flow rate at the first position of the coronary artery of interest.

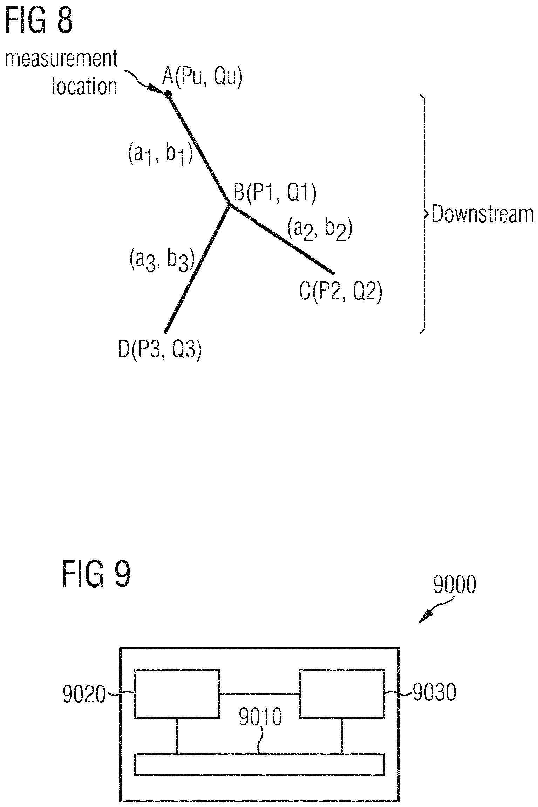

FIG. 8 schematically illustrates a still further exemplary lumped parameter model of a coronary artery of interest according to various examples. The only difference between FIG. 8 and FIG. 7 is that the entire segment of the coronary artery of interest upstream from the measurement location is completely missing. I.e., $(a_u, b_u)$ cannot be determined.

The FFR at the first position, i.e., point A, is measured, and *FFR = $P_u$/ aortic pressure.* As such, $P_u$ can be determined based on FFR and the *aortic pressure.* Further, $Q_u$ can be determined by solving equations (1) and (2).

As such, the respective pressure and flow rate at each of points B, C, and D can be determined iteratively as described in Scenario 2. Each of points B, C, and D may be the second position within a region of the coronary artery of interest arranged downstream with respect to the first position. The second position may be any position located in any of the branches AB, BC, and BD.

According to this disclosure, the *aortic pressure* may be either a population average value or a patient-specific value.

The equations (1) to (5) should be regarded just as a schematic, these are not the real equations which are solved. The real equations are much more complex, e.g., as disclosed in a non-patent literature - Itu, Lucian, et al. "A patient-specific reduced-order model for coronary circulation." 2012 9th IEEE international symposium on biomedical imaging (ISBI). IEEE, 2012.

Additionally or optionally, on top of the equations, there is a personalization framework, e.g., as disclosed in a non-patent literature - Sharma, Puneet, et al. "A framework for personalization of coronary flow computations during rest and hyperemia." 2012 Annual International Conference of the IEEE Engineering in Medicine and Biology Society. IEEE, 2012. Optionally or additionally, the method 3000 may further comprise comparing the respective values of the at least one hemodynamic index at the second position with corresponding values of the at least one hemodynamic index at the first position; and acquiring a further cardiac image depicting the entire coronary artery of interest, if said comparing indicates that one or more of the at least one hemodynamic index changes beyond a respective pre-defined threshold.

Optionally or additionally, the method 3000 may further comprise visualizing, in a cardiac image comprising the second position, the respective values of the at least one hemodynamic index at the position.

According to various examples, the multiple cardiac images may comprise multiple frames of an angiogram acquired using different angulations.

FIG. 9 is a block diagram of a computing device 9000 according to various examples. The computing device 9000 may comprise a processor 9020, a memory 9030, and an input/output interface 9010. The processor 9020 is configured to load program code from the memory 9030 and execute the program code. Upon executing the program code, the processor 9020 performs the method 3000 for processing multiple cardiac images depicting a coronary artery of interest.

The computing device 9000 may be embedded in or connected with an angiography device such as the C-arm machine 800. I.e., the angiography device comprising the computing device 9000 may be configured to perform the method 3000.

Referring to FIG. 1 again, the C-arm machine 800 may further comprise the computing device 9000 configured to perform the method 3000. The computing device 9000 may be the C arm control unit 810 and/or the control panel 850. I.e., the computing device 9000 may be embedded in or connected with the C-arm machine 800, and thereby the C-arm machine 800 may be also configured to perform the method 3000.

Summarizing, techniques have been described that facilitate determining at least one hemodynamic index when the region of interest for the computation/determination of the hemodynamic index is not entirely visible or not well visible in a single angiographic frame. Specifically, the techniques disclosed herein may facilitate determining at least one hemodynamic index based on individual angiographic frames with a limited field of view or incomplete field of view.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

For illustration, the disclosure is explained in detail based on X-ray angiography, the techniques disclosed herein can be also applied to angiography that may be done using scans instead of X-rays, such as CT (computed tomography) angiography or MRI (Magnetic resonance imaging) angiography.

Further, the approaches described herein may be used also when certain parts of the region of interest are not well visible on a certain frame, e.g., due to foreshortening, vessel overlap, etc.

In addition, the approaches described herein may be used also when the multiple cardiac images are obtained from different angiography views / acquired under different acquisition angles. Certain regions of interest may be recommended depending on the angulation of the acquisition (e.g. LM/pLAD/proximal LCx for the spider view). By combining the recommended regions of interest from the various angulations, a more accurate representation of the entire region of interest may be obtained.

Further, the approaches described herein may be used also when the multiple cardiac images are obtained from different imaging modalities: OCT / IVUS: highly accurate anatomical information on a single vessel/vessel segment or CCTA: entire coronary arterial tree modeled in 3D.

In certain clinical situations, panning may be performed to assess the status of the distal vessels, such as assessing the level of collateralization (presence and extent of collateral vessels). In such scenarios, additional information (beyond just the anatomy of the distal vessels) can be extracted from the clips after panning and used to better inform the algorithm for hemodynamic computation for the pre-panned images. Such additional information could represent the retrograde filling of the main vessel, the rate of flow of the contrast in the distal vessels etc.

According to this disclosure, a new acquisition mode can be defined which can automatically collect several limited field-of-view images/frames from different angulations such that these images can be optimally merged. This acquisition mode can utilize CCTA images to personalize the acquisition.

According to various examples, multiple frames can be used to generate a pseudo (i.e., synthetic) frame with a field of view large enough to capture the full coronary tree, by using deep learning techniques such as a conditional generative model. For example, the coronary lumen mask may be extracted from the multiple frames and then merged as described above. Next, the synthetic frame may be generated using as input the merged coronary lumen mask.

**Claims**

1. A computer-implemented method (3000), comprising:

   - obtaining (3100) multiple cardiac images (1100, 1200), each of the multiple cardiac images depicting a respective segment (1101, 1201) of a coronary artery of interest (1000);
   - determining (3200) a geometric structure of the coronary artery of interest (1000) based on the multiple cardiac images (1100, 1200);
   - determining (3300), based on the geometric structure, a lumped parameter model (4000) of the coronary artery of interest (1000);
   - determining (3400), based on the lumped parameter model (4000) of the coronary artery of interest (1000), respective values of at least one hemodynamic index at a position of the coronary artery of interest (1000).

2. The computer-implemented method (3000) of claim 1, said obtaining of the multiple cardiac images (1100, 1200) comprising:

   - determining, based on at least one frame at a beginning of an angiogram, at least one landmark (1001, 1002) associated with the coronary artery of interest (1000);
   - determining, among frames of the angiogram, a frame at which panning begins and a further frame at which the panning ends based on the at least one landmark (1001, 1002);
   - selecting, among the frames of the angiogram, the multiple cardiac images (1100, 1200) based on the frame and the further frame such that the multiple cardiac images (1100, 1200) pertain to the same cardiac phase and depicts the coronary artery of interest (1000).

3. The computer-implemented method (3000) of claim 1 or 2, the method (3000) further comprising:

   - merging the multiple cardiac images (1100, 1200) into a merged cardiac image;

   wherein said determining of the geometric structure of the coronary artery of interest (1000) is based on the merged cardiac image.

4. The computer-implemented method (3000) of claim 1 or 2, said determining of the geometric structure of the coronary artery of interest (1000) comprising:

   - determining, for each of the multiple cardiac images (1100, 1200), a respective segment (1101, 1201) of the geometric structure of the coronary artery of interest (1000) based on the respective one of the multiple cardiac images (1100, 1200);
   - merging the respective segments (1101, 1201) of the geometric structure of the coronary artery of interest into the geometric structure of the coronary artery of interest (1000).

5. The computer-implemented method (3000) of any one of the preceding claims, further comprising:

   - obtaining at least one measurement associated with the at least one hemodynamic index at a further position (A) within a region of the coronary artery of interest (1000) arranged upstream with respect to the position;

   wherein said determining of the respective values of the at least one hemodynamic index at the position is further based on the at least one measurement at the further position (A) .

6. The computer-implemented method (3000) of claim 5, wherein the at least one hemodynamic index comprise a blood pressure and a blood flow rate, and said determining of the respective values of the at least one hemodynamic index at the position comprises:

   - determining, based on the at least one measurement, both the blood pressure and the blood flow rate at the further position (A) of the coronary artery of interest (1000);
   - determining, using the lumped parameter model (4000) of the coronary artery of interest (1000), coefficients characterizing a total energy loss of blood flow passing from the further position (A) to the position based on the geometric structure of a segment of the coronary artery of interest (1000) between the further position (A) and the position;
   - determining, based on both the blood pressure and the blood flow rate at the further position (A) of the coronary artery of interest (1000) and the coefficients, the respective values of the at least one hemodynamic index at the position.

7. The computer-implemented method (3000) of claim 6, wherein the at least one measurement comprises both the blood pressure and the blood flow rate at the further position (A) of the coronary artery of interest (1000).

8. The computer-implemented method (3000) of claim 6, wherein the at least one measurement comprises a blood pressure drop between the root of the aorta and the further position (A), and the geometric structure of the coronary artery of interest (1000) comprises a further segment of the coronary artery of interest between the root of the aorta and the further position (A), said determining of both the blood pressure and the blood flow rate at the further position (A) of the coronary artery of interest (1000) comprising:

   - obtaining the central aortic blood pressure at the root of the aorta;
   - determining, using the lumped parameter model (4000) of the coronary artery of interest (1000), further coefficients characterizing a further total energy loss of blood flow passing from the root of the aorta to the further position (A) based on the geometric structure of the further segment of the coronary artery of interest (1000) between the root of the aorta and the further position (A);
   - determining, based on the blood pressure drop, the central aortic blood pressure, and the further coefficients, both the blood pressure and the blood flow rate at the further position (A) of the coronary artery of interest (1000).

9. The computer-implemented method (3000) of claim 6, said determining of both the blood pressure and the blood flow rate at the further position (A) of the coronary artery of interest (1000) comprising:

- obtaining the central aortic blood pressure at the root of the aorta, wherein the at least one measurement comprises a fractional flow reserve, FFR, measured at the further position (A) and relative to the central aortic blood pressure;
- determining, based on the central aortic blood pressure, and the FFR, both the blood pressure and the blood flow rate at the further position (A) of the coronary artery of interest (1000).

10. The computer-implemented method (3000) of any one of claims 5 to 9, further comprising:

- comparing the respective values of the at least one hemodynamic index at the position with corresponding values of the at least one hemodynamic index at the further position (A);
- acquiring a further cardiac image depicting the entire coronary artery of interest (1000), if said comparing indicates that one or more of the at least one hemodynamic index changes beyond a respective pre-defined threshold.

11. The computer-implemented method (3000) of any one of claims 5 to 10, where the at least one measurement associated with the at least one hemodynamic index at the further position (A) is obtained using a pressure wire.

12. The computer-implemented method (3000) of any one of claims 5 to 11, wherein the at least one measurement comprises measurement obtained using at least one of the following imaging machines: a C-arm X-ray machine (800), an intravascular ultrasound machine, an optical coherence tomography machine, and a coronary computed tomography angiography machine.

13. The computer-implemented method (3000) of any one of the preceding claims, further comprising:

- visualizing, in a cardiac image comprising the position, the respective values of the at least one hemodynamic index at the position.

14. A computing device (9000), the device comprising a processor (9020) and a memory (9030), wherein upon loading and executing program code from the memory (9030), the processor (9020) is configured to perform the method (3000) of any one of the preceding claims.

15. An angiography device (800) comprising the computing device (9000) of claim 14.

FIG 1

# FIG 2

# FIG 3

3000

obtaining multiple cardiac images, each of the multiple cardiac images depicting a respective segment of a coronary artery of interest ~3100

determining a geometric structure of the coronary artery of interest based on the multiple cardiac images ~3200

determining, based on the geometric structure, a lumped parameter model of the coronary artery of interest ~3300

determining, based on the lumped parameter model of the coronary artery of interest, respective values of at least one hemodynamic index at a position of the coronary artery of interest ~3400

## FIG 4

## FIG 5

## FIG 6

## FIG 7

inlet (aortic pressure)

$(a_u, b_u)$ Upstream

$A(Pu, Qu)$

measurement location

$(a_1, b_1)$

$B(P1, Q1)$

$(a_2, b_2)$

Downstream

$(a_3, b_3)$

$C(P2, Q2)$

$D(P3, Q3)$

# FIG 8

measurement
location

A(Pu, Qu)

$(a_1, b_1)$

B(P1, Q1)

$(a_2, b_2)$

$(a_3, b_3)$

C(P2, Q2)

Downstream

D(P3, Q3)

# FIG 9

9000

9020

9010

9030

Europäisches
Patentamt

European
Patent Office

Office européen
des brevets

## EUROPEAN SEARCH REPORT

Application Number

EP 22 21 7379

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/100522 A1 (NICKISCH HANNES [DE] ET AL) 8 April 2021 (2021-04-08) | 1,3,4, 13,14 | INV. G06T7/00 |
| Y | * claim 13 * <br> * paragraphs [0029] – [0032], [0072], [0015], [0066], [0034], [0072], [0070], [0065], [0066], [0022], [0025] – [0026] * | 2,5-11 | G16H50/30 |
| X | US 2015/272448 A1 (FONTE TIMOTHY A [US] ET AL) 1 October 2015 (2015-10-01) | 1,12-15 | |
| Y | * claim 18 * <br> * figures 12,15 * <br> * paragraphs [0153], [0049] * <br> * figures 20-22 * <br> * paragraphs [0309], [0363] * <br> * figure 23 * | 2,5-11 | |
| Y | US 2015/324962 A1 (ITU LUCIAN MIHAI [RO] ET AL) 12 November 2015 (2015-11-12) <br> * paragraph [0056] * <br> * paragraph [0030] * | 5-11 | TECHNICAL FIELDS SEARCHED (IPC) |
| A | JP 6 738406 B2 (KONIKLIJKE PHILIPS N.V.) 12 August 2020 (2020-08-12) <br> * paragraph [0023] * | 12 | G06T G16H |

The present search report has been drawn up for all claims

| Place of search | Date of completion of the search | Examiner |
|---|---|---|
| Munich | 5 June 2023 | Winkler, Gregor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 22 21 7379

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2023

| Patent document cited in search report | | | Publication date | Patent family member(s) | | | Publication date |
|---|---|---|---|---|---|---|---|
| US 2021100522 | A1 | | 08-04-2021 | CN | 110636798 | A | 31-12-2019 |
| | | | | EP | 3384850 | A1 | 10-10-2018 |
| | | | | EP | 3606437 | A1 | 12-02-2020 |
| | | | | JP | 7203754 | B2 | 13-01-2023 |
| | | | | JP | 2020512885 | A | 30-04-2020 |
| | | | | US | 2021100522 | A1 | 08-04-2021 |
| | | | | WO | 2018185038 | A1 | 11-10-2018 |
| US 2015272448 | A1 | | 01-10-2015 | EP | 3127026 | A1 | 08-02-2017 |
| | | | | JP | 6378779 | B2 | 22-08-2018 |
| | | | | JP | 6634123 | B2 | 22-01-2020 |
| | | | | JP | 6905574 | B2 | 21-07-2021 |
| | | | | JP | 2017512577 | A | 25-05-2017 |
| | | | | JP | 2018196742 | A | 13-12-2018 |
| | | | | JP | 2020044375 | A | 26-03-2020 |
| | | | | US | 9087147 | B1 | 21-07-2015 |
| | | | | US | 2015272448 | A1 | 01-10-2015 |
| | | | | US | 2015324545 | A1 | 12-11-2015 |
| | | | | US | 2016180055 | A1 | 23-06-2016 |
| | | | | US | 2017220760 | A1 | 03-08-2017 |
| | | | | US | 2017329930 | A1 | 16-11-2017 |
| | | | | US | 2022406470 | A1 | 22-12-2022 |
| | | | | WO | 2015153362 | A1 | 08-10-2015 |
| US 2015324962 | A1 | | 12-11-2015 | CN | 105078440 | A | 25-11-2015 |
| | | | | EP | 2942006 | A1 | 11-11-2015 |
| | | | | ES | 2641688 | T3 | 13-11-2017 |
| | | | | US | 2015324962 | A1 | 12-11-2015 |
| JP 6738406 | B2 | | 12-08-2020 | CN | 107851463 | A | 27-03-2018 |
| | | | | EP | 3332339 | A1 | 13-06-2018 |
| | | | | JP | 6738406 | B2 | 12-08-2020 |
| | | | | JP | 2018533383 | A | 15-11-2018 |
| | | | | US | 2018211729 | A1 | 26-07-2018 |
| | | | | WO | 2017021201 | A1 | 09-02-2017 |

EPO FORM P0459

For more details about this annex : see Official Journal of the European Patent Office, No. 12/82

## REFERENCES CITED IN THE DESCRIPTION

**Patent documents cited in the description**

- US 11051779 B2

**Non-patent literature cited in the description**

- Comparison of fractional flow reserve based on computational fluid dynamics modeling using coronary angiographic vessel morphology versus invasively measured fractional flow reserve. *The American journal of cardiology,* 2016, vol. 117 (1), 29-35
- **ÇIMEN, SERKAN et al.** Reconstruction of coronary arteries from X-ray angiography: A review. *Medical image analysis,* 2016, vol. 32, 46-68
- **ANDRIOTIS, ADAMANTIOS et al.** A new method of three-dimensional coronary artery reconstruction from X-ray angiography: Validation against a virtual phantom and multislice computed tomography. *Catheterization and cardiovascular interventions,* 2008, vol. 71 (1), 28-43
- **ITU, LUCIAN et al.** A machine-learning approach for computation of fractional flow reserve from coronary computed tomography. *Journal of applied physiology,* 2016, vol. 121 (1), 42-52
- A patient-specific reduced-order model for coronary circulation. **ITU, LUCIAN et al.** 2012 9th IEEE international symposium on biomedical imaging (ISBI). IEEE, 2012
- A framework for personalization of coronary flow computations during rest and hyperemia. **SHARMA, PUNEET et al.** 2012 Annual International Conference of the IEEE Engineering in Medicine and Biology Society. IEEE, 2012